# EUROPEAN PATENT APPLICATION

(11) **EP 4 434 935 A1**
(43) Date of publication of application: **25.09.2024**
(21) Application number: 23163012.0
(22) Date of filing: 21.03.2023
(51) Int. Cl.: C01B 3/08, C07C 1/12

(54) **METHOD AND EQUIPMENT FOR IN SITU WATER REMOVAL AND HYDROGEN PRODUCTION DURING CO2 HYDROGENATION REACTION**

(71) Applicant: Lietuvos Energetikos Institutas, 44403 Kaunas (LT)
(72) Inventor: MILCIUS, Darius, 51310 Kaunas (LT); VARNAGIRIS, Sarunas, 49286 Kaunas (LT); URBONAVICIUS, Marius, 54306 Vijukai, Kauno r. sav. (LT); GIMZAUSKAITE, Dovile, 09206 Vilnius (LT); AIKAS, Mindaugas, 45311 Kaunas (LT); USCILA, Rolandas, 44461 Kaunas (LT); TAMOSIUNAS, Andrius, 53458 Virbaliskiai, Kauno r. sav. (LT)
(74) Representative: Pranevicius, Gediminas

(57) **Abstract**

The presented invention relates to the method and equipment of water molecules removal from CO₂ + H₂ mixtures and biogases with the following production of H₂ and reaction by-products Bayerite - Al(OH)₃, Na₂CO₃•H₂O thermonatrite and useful heat. The method comprising: activating the aluminium scrap in glow discharge plasma in the presence of 3-10 Pa of hydrogen, or argon, or hydrogen and argon mixtures with the best Ar:H₂ ratio at 70:30 as residual gasses for 10-60 min with the DC power density up to 10-30 W/cm² while keeping the temperature in the range of 60-110 ⁰C during the plasma treatment process; placing hydrophilic activated aluminium (5) in a layered structure of the reaction chamber (1) between layers (4) enriched with NaOH particles acting as hydrogen generation promoters; introducing CH₄ + H₂O mixture into the reaction chamber (1) with hydrogen; obtaining the CH₄+H₂ mixture (7) and the reaction by-products Bayerite - Al(OH)₃ and Na2CO₃•H₂O thermonatrite; separating the H₂ from the CH₄ + H₂ mixture (7).

## Description

### TECHNICAL FIELD

The presented invention relates generally to the removal of water molecules from CO₂ + H₂ mixtures and biogases with the following production of H₂ and reaction by-products Bayerite - Al(OH)₃, Na₂CO₃•H₂O thermonatrite and useful heat.

### BACKGROUND ART

Reversible CO₂ hydrogenation reaction (Sabatier reaction) and biogases requires *in situ* water removal to get higher methane yield and selectivity.

The four main methods for the removal of water and water vapour from CO₂ + H₂ mixtures and biogases are mainly used today by industry: 1) condensation (refrigeration); 2) adsorption drying; 3) absorption drying, and 4) use of special membranes.

*Condensation based methods.* Using this approach, the gas mixtures are cooled down at atmospheric pressure to condense the excessive water vapour. The condensed water droplets are then separated using: 1) demisters, in which the water droplets are separated using a wired mesh; 2) cyclone separators, in which the water droplets are separated using centrifugal force, equal to hundreds of times the force of gravity; 3) moisture traps, in which the condensation occurs by the expansion of biogas, and subsequently a drop in temperature that condenses the water vapour; and/or 4) water taps in the gas pipe, used for the collection of condensed water (Golmakani A, Nabavi SA, Wadi B, Manovic V. Advances, challenges, and perspectives of biogas cleaning, upgrading, and utilisation. Fuel. Volume 317, 1 June 2022, 123085 https://doi.org/10.1016/j.fuel.2021.123085).

*Adsorption drying.* Using this approach water molecules were adsorbed using solid adsorbents with high BET, such as Silica based, for example Silica-NH₂-MAS; Silica-NH₂-PSS; Silica-NH₂-PAAS (Pei L, Zhang L. Preparation and selective adsorption of core-shell desiccant for heat and moisture recovery. Colloids Surfaces A Physicochem Eng Asp 2012;406: 68-74. https://doi.org/10.1016/j.colsurfa.2012.04.052), activated alumina (Solomon I, Anam Ribeiro, Santos JC, Loureiro JM, Rodrigues AE, Sandu I, et al. Adsorption equilibrium of water vapor on activated carbon and alumina and carbon and alumina impregnated with hygroscopic salt. Turkish J Chem 2013;37: 358-65), activated carbons without and with impregnation with hydroscopic salts (Tso CY, Chao CYH. Activated carbon, silica-gel and calcium chloride composite adsorbents for energy efficient solar adsorption cooling and dehumidification systems. Int J Refrig 2012; 35:1626-38. https://doi.org/10.1016/j. ijrefrig.2012.05.007), polymer structures impregnated with hygroscopic salts (Lee J, Lee D-Y. Sorption characteristics of a novel polymeric desiccant. Int J Refrig 2012; 35:1940-9. https://doi.org/10.1016/j.ijrefrig.2012.07.009) and metal organic frameworks (Seo YK, Yoon JW, Lee JS, Hwang YK, Jun CH, Chang JS, et al. Energy-efficient dehumidification over hierachically porous metal-organic frameworks as advanced water adsorbents. Adv Mater 2012;24:806-10. https://doi.org/ 10.1002/adma.201104084*).*

*Absorption drying.* It could be realized using hydroscopic salts and salts should be continuously replaced after saturation with water molecules (Persson M, Jonsson O, Biogas Wellinger A. Upgrading To Vehicle Fuel Standards and Grid. 2007*).* The same approach could be realized using glycol as absorption media.

Most of the novel developments are related with the use of hybrid materials containing both catalyst (Cu/UGSO, UGSO - UpGraded slag oxide) and hydrophilic adsorbent (13X zeolite) for in-situ water removal for gas mixtures (Desgagnes A, Iliuta M.C. Intensification of CO2 hydrogenation by in-situ water removal using hybrid catalyst-adsorbent materials: Effect of preparation method and operating conditions on the RWGS reaction as a case study. Chemical Engineering Journal 454 (2023) 140214. https://doi.org/10.10167j.cej.2022.140214*)* in sorption-enhanced (SE) reaction process, which allows the reactors to be operated at much lower temperatures to achieve the same conversion of reactants.

### SUMMARY OF THE INVENTION

The technology and equipment described herein allows to transform *in situ* water molecules from CO₂ + H₂ mixtures and biogases to pure hydrogen and aluminium and water reaction by-products. The produced hydrogen could be directed back to the reaction chamber and produced heat used for other purposes.

The invention relates to the method of removal of water molecules using the direct reaction between CO₂ + H₂ mixtures or biogases with plasma activated aluminium. Aluminium scrap activation is performed in glow discharge plasma in the presence of 3-10 Pa of hydrogen with purity not less than 99.00 % or argon with purity not less than 99.00 %, or hydrogen and argon mixtures at the best Ar:H₂ proportion 70:30 as residual gasses for 10-60 min treatment in plasma. The temperature during the plasma treatment of aluminium scrap process is 60-110 ⁰C. The hydrophilic activated aluminium is placed in a reaction chamber in layered structure between the layers of NaOH particles. Stainless steel mesh with pores diameters 1 mm was used at the inlet and outlet of reaction molecules to prevent aluminium and reaction by-products migration from the innovative reaction chamber. Two modifications of equipment setup could be realized using the same developed innovative reaction chamber:
1) CH₄ + H₂O mixture directly enters the chamber and hydrogen; Bayerite - Al(OH)₃, Na₂CO₃•H₂O thermonatrite; heat was produced and CH₄ + H₂ leaves the reaction chamber; H₂ could be separated from CH₄ + H₂ mixture and further used in reactions with CO₂ again; and
2) CH₄ + H₂O enters water vapour condensation equipment, condensed water enters the reaction chamber and hydrogen, Bayerite - Al(OH)₃, Na₂CO₃•H₂O thermonatrite, the reaction heat was produced and H₂ leaves the reaction chamber and could be used in the Sabatier reactor with the reaction CO₂ again. The main novelty of the invention is related to the development of the reaction chamber filled with hydrophilic activated aluminium, and glass wool layers enriched with layers of NaOH particles to enhance chemical reactions. Stainless steel mesh with pores diameters up to 1 mm was used at the inlet and outlet of developed innovative reaction chamber to prevent the migration of reaction materials from the chamber.

### BRIEW DESCRIPTION OF THE DRAWINGS

The presented invention is described with the reference to the accompanying drawings:
Fig.1 depicts a schematic view illustration of innovative reaction chamber;
Fig. 2 depicts a schematic view illustration of the process for water molecules removal and production of hydrogen;
Fig. 3 depicts a schematic view illustration of the process for water molecules removal and production of hydrogen;
Fig.4 depicts scanning electron images of initial activated aluminium scrap at two magnifications and X-ray diffraction pattern which corresponds to pure aluminium;
Fig.5 depicts scanning electron microscope images of water and activated aluminium reaction by-products and X-ray diffraction pattern which corresponds to Bayerite - Al(OH)₃, Na₂CO₃•H₂O thermonatrite.

### DETAILED DESCRIPTION OF THE INVENTION

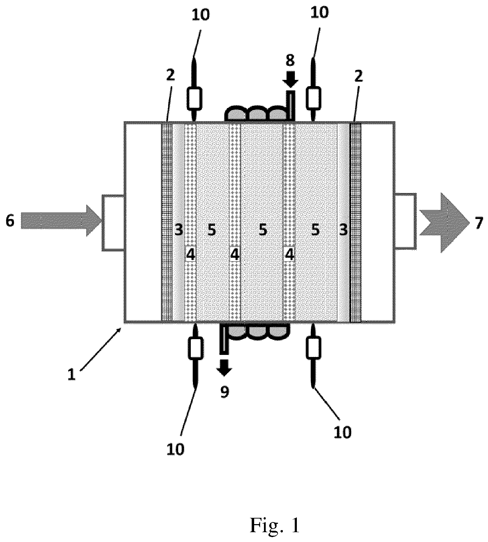

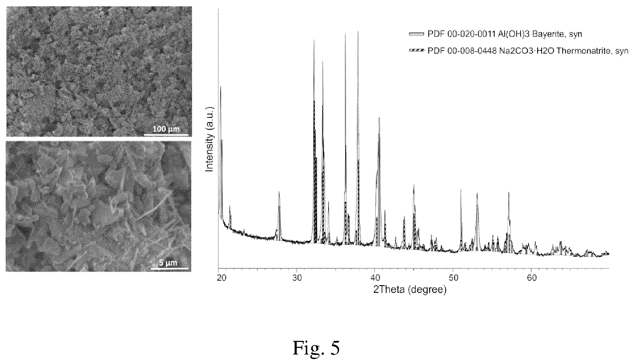

Schematic views of the present invention are presented in Fig. 1-5. The invention relates to the method of removal of water molecules using the direct reaction between CO₂ + H₂ mixtures or biogases with plasma activated aluminium. The activation of the aluminium scrap is performed in glow discharge plasma in the presence of 3-10 Pa of hydrogen with purity not less than 99.00 %, or argon with purity not less than 99.00 %, or hydrogen and argon mixtures with the best Ar:H₂ ratio at 70:30 as residual gasses for 10-60 min treatment in plasma. The temperature during the plasma treatment of aluminium scrap process is 60-110 °C.

The removal of water molecules from CO₂ + H₂O and biogases occurs in an innovative reaction chamber 1 which comprises hydrophilic activated aluminium 5 placed in the chamber 1 in layered structure between layers 4 enriched with NaOH particles as hydrogen generation promoters. Stainless steel mesh 2 with pores diameters less or equal to 1 mm and glass wool layers 3 were used at the inlet and outlet of the reaction chamber 1 to prevent escape of reaction products from the chamber.

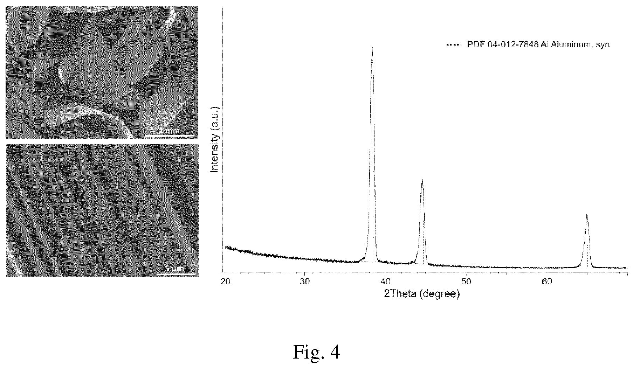

Detailed description of water molecules removal:
Aluminium scrap, which consist of non-uniform flake like particles with the dimensions more than 1 mm in length and width and consist of almost pure crystalline aluminium, as could be seen from x-ray diffraction pattern (Fig. 4), is activated in glow discharge plasma in the presence of 3-10 Pa of hydrogen with purity not less than 99.00 %, or argon with purity not less than 99.00 %, or hydrogen and argon mixtures with the best Ar:H₂ ratio at 70:30 as residual gasses for 10-60 min treatment in plasma with the DC power density up to 10 - 30 W/cm². Temperature is 60-110 ⁰C during the plasma process.

Plasma activated hydrophilic aluminium 5 is placed in the reaction chamber 1 in layered structure between layers 4 enriched with NaOH particles. The proportion of layers thickness between layers 4 enriched with NaOH particles, which act as reaction promoters between plasma activated aluminium and water molecules, and activated aluminium layer 5 is 1:5 by volume. A stainless steel mesh 2 with pores diameters 1 mm and glass wool layers 3 were used at the inlet and outlet of reaction molecules to prevent aluminium 5 and reaction by-products Bayerite - Al(OH)₃, Na₂CO₃•H₂O thermonatrite migration from the reaction chamber. Reaction gasses 6, which consist of CH₄+H₂O, enter the reactor chamber 1, and CH₄+H₂ mixture 7 was produced.

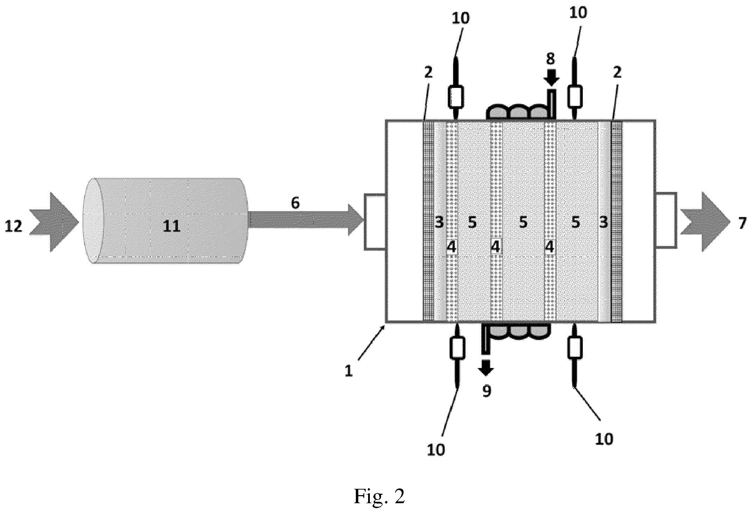

The similar reaction chamber could be connected using two configurations shown in Fig. 2-3. In case of Fig. 2 configuration, initial reaction gasses CH₄ + H₂O, in Fig. 2 shown as 6, which leaves Sabatier reactor 11, enters the reaction chamber 1 where reaction between activated aluminium 5 and water molecules takes place and mixture of CH₄ + H₂ gases 7, the reaction by-products Bayerite - Al(OH)₃, Na₂CO₃•H₂O thermonatrite and useful heat are produced. No preliminary water condensation is needed in this case.

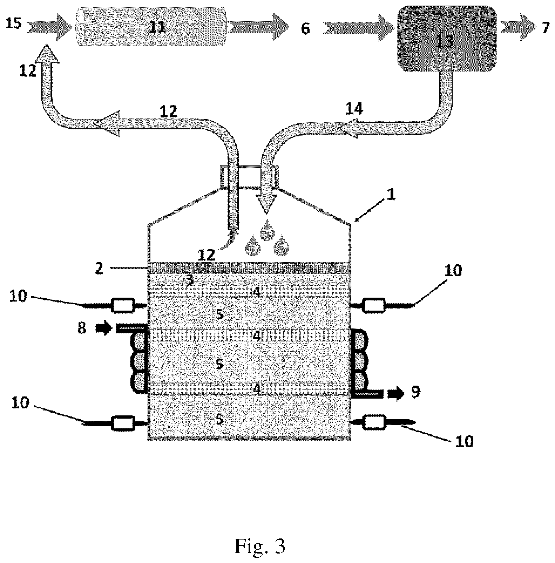

In case water condensation equipment is present (Fig. 3), H₂O molecules 15 together with CO₂ enter Sabatier reactor 11 and CH₄ + H₂O water vapour 6 produced. The vapour 6 enters water vapour condenser 13, and condensed water 14 enters the reaction chamber 1 where reaction between activated aluminium 5, layers 4 of NaOH particles, and water molecules 14 takes place, and pure H₂ gas and reaction by-products Bayerite - Al(OH)₃, Na₂CO₃•H₂O thermonatrite are produced. Whereas oxygen is captured in the reaction by-products. Produced CH₄ gas 7 could be used for final applications and produced H₂ gas 12 could be separated and supplied to the Sabatier reactor 11 in a close cycle system for CO₂ + H₂ reactions again. Stainless steel mesh 2 with pores diameters of 1 mm together with glass wool layers 3 were used at the inlet and outlet of reaction molecules to prevent migration of aluminium 5 and reaction by-products Bayerite - Al(OH)₃, Na₂CO₃•H₂O thermonatrite from the reaction chamber. NaOH layers 4 were used as reaction promoters. The reaction by-products Bayerite - Al(OH)₃, Na₂CO₃•H₂O and useful heat are produced during reactions between activated aluminium 5 and water molecules.

The received H₂ gas 12 in case Fig. 3 configuration or separated H₂ from the received CH₄ + H₂ mixture in case Fig. 2 could be used in Sabatier reactor 11 for reaction with CO₂ again. The solid state reaction by-products for the both cases are the same and consist of Bayerite - Al(OH)₃, Na₂CO₃•H₂O thermonatrite.

As the reaction between activated aluminium and water molecules was exothermic, useful heat is produced and removed from the reaction chamber using similar heat exchange fluid 8 (Fig. 2-3).

Heat management in all cases (Fig. 1-3) was realized using a heat exchange fluid 8 with Tmin, ⁰C, which heats up and dissipate produced heat at exit where heat exchange fluid 9 Tmax, ⁰C and could be used for further applications. Four thermocouples 10 were installed in different places of the innovative chamber 1. Water could be used as a heat exchange fluid in all cases.

As the reaction is exothermic, from 1 kg of aluminium and 2 kg of water molecules could be produced up to 0.11 kg of H₂ and 4.3 kWh of useful heat for both configurations.

## Claims

1. A method for *in situ* removal of water molecules from CO₂ + H₂O and biogases and hydrogen production during CO₂ hydrogenation reaction, **characterized by** comprising:
- activating the aluminium scrap in glow discharge plasma in the presence of 3-10 Pa of hydrogen with purity not less than 99.00 %, or argon with purity not less than 99.00 %, or hydrogen and argon mixtures with the best Ar:H₂ ratio at 70:30 as residual gasses for 10-60 min with the DC power density up to 10-30 W/cm² while keeping the temperature in the range of 60-110 ⁰C during the plasma treatment process;
- placing the hydrophilic activated aluminium (5) in a layered structure of the reaction chamber (1) between layers (4) enriched with NaOH particles as hydrogen generation promoters,
- introducing CH₄ + H₂O mixture directly into the reaction chamber (1) with hydrogen; obtaining the CH₄+H₂ mixture (7), which leaves the reaction chamber (1), and the reaction by-products Bayerite - Al(OH)₃ and Na₂CO₃•H₂O thermonatrite;
- separating the H₂ from the CH₄ + H₂ mixture (7), and introducing the H₂ into the reaction with CO₂ again,
- capturing the oxygen in reaction by-products Bayerite - Al(OH)₃ and Na₂CO₃•H₂O thermonatrite.

2. An equipment for *in situ* removal of water molecules from CO₂ + H₂O and biogases and hydrogen production during CO₂ hydrogenation reaction, the equipment comprising a reaction chamber (1), wherein the reaction chamber (1) comprises a layered structure consisting of layers (4) enriched with NaOH particles as hydrogen generation promoters, between which layers the hydrophilic activated aluminium (5) is placed, and stainless steel meshes (2) with pores diameters less or equal to 1 mm together with glass wool layers (3) are placed at the inlet (6) and outlet (7) of gases to prevent the aluminium and reaction by-products Bayerite - Al(OH)₃, Na₂CO₃•H₂O thermonatrite migration from the reaction chamber.
